# EUROPÄISCHE PATENTSCHRIFT

(11) **EP 2 272 546 B1**
(45) Veröffentlichungstag und Bekanntmachung des Hinweises auf die Patenterteilung: **07.08.2019**
(21) Anmeldenummer: 10166496.9
(22) Anmeldetag: 18.06.2010
(51) Int. Cl.: A61L 31/02, A61L 31/14, A61L 31/16

(54) **Implantat und Verfahren zur Herstellung desselben**
Implant and method for manufacturing the same
Implant et son procédé de fabrication

(30) Priorität: 07.07.2009 US 223414 P
(43) Veröffentlichungstag der Anmeldung: 12.01.2011
(73) Patentinhaber: BIOTRONIK AG, 8180 Bülach (CH)
(72) Erfinder: Bayer, Ullrich, 18211 Admannshagen-Bargeshagen (DE); Harder, Claus, 91080 Uttenreuth (DE); Burean. Elisabeta, 91052 Erlangen (DE)
(74) Vertreter: Keck, Hans-Georg

(56) Entgegenhaltungen:
- WO-A1-01/45862
- WO-A1-02/060506
- WO-A2-2007/095549
- US-B1- 6 558 733
- US-B2- 6 558 773

## Beschreibung

Die vorliegende Erfindung betrifft ein Verfahren zur Herstellung eines Implantats, insbesondere einer intraluminalen Endoprothese, mit einem Körper enthaltend metallisches Material, vorzugsweise Eisen, insbesondere eine Eisenlegierung, umfassend die folgenden Schritte:
a) Bereitstellen des Körpers des Implantats,
b) Herstellung einer Porenstruktur mit zumindest teilweise geschlossenen Poren in einem Teil des oberflächennahen Gefüges des Implantatkörpers,
c) Einlagerung von NOₓ in die Hohlräume der Porenstruktur,
dadurch gekennzeichnet, dass die Porenstruktur mittels Wärmebehandlung des Implantatkörpers in einer Sauerstoff enthaltenden und/oder Kohlenstoff enthaltenden Atmosphäre hergestellt wird, sowie ein Implantat, insbesondere eine intraluminalen Endoprothese, mit einem Körper enthaltend ein degradierbares metallisches Material, vorzugsweise Eisen, dadurch gekennzeichnet, dass der Implantatkörper eine Porenstruktur mit zumindest teilweise geschlossenen Poren in einem Teil seines oberflächennahen Gefüges aufweist, welche in ihren Hohlräumen NOx enthält.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die beispielsweise eine Entzündung (Inflammation) des behandelten Gefäßes oder eine nekrotische Gefäßveränderung begünstigen und die zu einem allmählichen Zuwachsen des Stents durch Bildung von Plaques führen können. Im schlimmsten Fall kann diese Gefäßveränderung einen Gefäßverschluss bedingen.

Derzeit werden häufig Implantate eingesetzt, welche aus einem biodegradierbaren Werkstoff gefertigt sind. Diese erweisen sich als vorteilhaft hinsichtlich der oben angegebenen Probleme mit Implantaten.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Eisen, Mangan und/oder Wolfram, insbesondere eine Eisenbasislegierung (im Folgenden kurz: Eisenlegierung).

Als Implantatmaterialien können auch andere metallische Werkstoffe eingesetzt werden. Implantate mit einer Eisenlegierung, insbesondere eisenhaltige Stents, sind besonders kostengünstig und einfach herstellbar.

Unter Biodegradation werden chemische, insbesondere hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Es ist wünschenswert, dass die oben beschriebene, eine Inflammation begünstigende Wirkung von Implantaten in Zukunft möglichst vermieden oder verringert wird, da hierdurch die Wirksamkeit des Implantats verringert wird und weitere Schädigungen des behandelten Organismus hervorgerufen werden können.

In der Druckschrift US 6,613,432 B2 (WO014/5862) wird eine Plasmabehandlung in einem Plasma mit Stickstoff und Sauerstoff enthaltenden Molekülen zur Reduktion der inflammatorischen Reaktion sowie zur Vermeidung einer Restenose vorgeschlagen. Die Plasmabehandlung dauert nur über einen kurzen Zeitraum an, bspw. weniger als etwa fünf Minuten. Eine derartige Plasmabehandlung von Implantaten ist jedoch gerätetechnisch vergleichsweise aufwändig und zudem mit einem erhöhten manuellen Aufwand verbunden. Filigrane Bauteile wie Stents bedürfen bei den Chargierungsvorgängen einer ausgeprägten Feinmotorik, die derzeit durch automatische Handlingsysteme nicht erzielbar ist. Weiterhin führen derartige Plasmabehandlungen nur an der unmittelbaren Oberfläche, d.h. bis in eine Tiefe von wenigen nm, zu einer Beladung mit antiinflammatorisch wirksamen Materialien. Erreicht der spätere Degradationsprozess weiter innen liegende Zonen des Implantates, welche durch die Plasmabehandlung nicht erreicht wurden, liegt keine antiinflammatorische Wirkung mehr vor. Diese innen liegenden Zonen werden bei der Degradation unter Umständen bereits zu einem sehr frühen Zeitpunkt nach dem Einsetzen des Implantats in das zu behandelnde Körperteil erreicht, nach dem weiterhin inflammatorische Reaktionen unterdrückt werden sollen. Ferner ist davon auszugehen, dass aus einer Plasmabeschichtung nur relativ geringer Mengen aktiver, die Inflammation senkende Substanzen an die Gefäßwand abgegeben werden können.

Folglich besteht die Aufgabe der vorliegenden Erfindung darin, ein einfaches und kostengünstiges Verfahren zur Herstellung eines aus metallischem Material gefertigten Implantats anzugeben, welches nach dem Einbringen in den behandelten Organismus eine verlängerte antiinflammatorische Wirkung des Implantats begünstigt. Dabei sollen keine festen oder flüssigen Wirkstoffe freigesetzt werden. Zudem soll die Wechselwirkung mit dem Material des Implantatkörpers oder deren Reaktionsprodukten minimiert werden. Entsprechend besteht die Aufgabe der Erfindung außerdem darin, ein derartiges Implantat zu schaffen.

Die obige Aufgabenstellung wird gelöst durch ein Verfahren zur Herstellung eines Implantates mit einem Körper enthaltend metallisches Material, umfassend die folgenden Schritte:
a) Bereitstellen des Körpers des Implantats,
b) Herstellung einer Porenstruktur mit zumindest teilweise geschlossenen Poren in einem Teil des oberflächennahen Gefüges des Implantatkörpers,
c) Einlagerung von NOₓ in die Hohlräume der Porenstruktur,
**dadurch gekennzeichnet,** dass die Porenstruktur mittels Wärmebehandlung des Implantatkörpers in einer Sauerstoff enthaltenden und/oder Kohlenstoff enthaltenden Atmosphäre hergestellt wird.

Bei der vorliegenden Erfindung umfasst der Körper des Implantats mindestens einen Teil des Implantats, vorzugsweise den Hauptteil des Implantats, welcher die mechanische Integrität des Implantats bewirkt.

Das erfindungsgemäße Verfahren erzeugt in Verfahrensschritt b) eine oberflächennahe Porenstruktur mit Poren (im Folgenden auch als Leerstellen, Mikrokavernen oder Blasen bezeichnet). Diese Porenstruktur beinhaltet zumindest teilweise geschlossene Poren. In die Hohlräume der in Schritt b) erzeugten Porenstruktur erfolgt anschließend eine Einlagerung von Stickstoffoxiden (auch als Stickoxide, nitrose Gase oder NOₓ bezeichnet) aufgrund von Chemisorption, Adsorption oder Gasfüllung. Diese Verbindungen werden im Laufe des Verbleibens des Implantats in dem behandelten Organismus, insbesondere bei einer Degradation der oberflächennahen Bereiche des Implantats, freigesetzt und üben auf das umgebende Zellgewebe positive biologische Effekte aus. NOₓ wirken aufgrund ihrer Eigenschaft als Radikalfänger insbesondere antiinflammatorisch, aber auch antibakteriell und zytostatisch. Eine über einen längeren Zeitraum vorhandene konstante Freisetzung auch von kleineren Dosen von NOₓ ist vor allem im vaskulären Bereich äußerst wünschenswert.

Die Einlagerung von NOₓ erfolgt vorzugsweise in die Leerstellen, welche durch das vorangegangene Austreiben der COₓ-Verbindungen im oberflächennahen Gefüge entstanden sind. Die so erzeugten Poren sind demnach nicht als makroskopische Poren zu verstehen sondern stellen eher mikroskopische Strukturen mit einem Durchmesser von weniger als 1 µm dar. Da der Molekülradius der NOₓ-Verbindungen kleiner als der der COₓ-Verbindungen ist, wird die Wiederbelegung dieser Leerstellen aus kristallografischen Aspekten begünstigt. Eine besonders hohe Beladung mit NOₓ wird erreicht, wenn diese unmittelbar nach der Glühbehandlung erfolgt. Andernfalls erfolgt eine alsbaldige Rekombination der Leerstellen mit dem übrigen Gefüge. Dies verschlechtert die Diffusion des NOₓ und bewirkt somit eine geringere Absolutbeladung mit NOₓ.

Die Leerstellen selbst befinden sich vor allem in der Nähe der Korngrenzen, da einerseits die hier vorliegende Korngrenzendiffusion für NOₓ größer ist als die Diffusion über das Korninnere, andererseits aber auch die Defektdichte und somit auch das Lösungsvermögen für Fremdverbindungen größer ist. Hieraus folgt, dass die Beladung mit NOₓ vorzugsweise dadurch erhöht werden kann, indem in dem Gefüge überwiegend kleine Körner vorgesehen werden, welche eine höhere Korngrenz-Dichte aufweisen.

Unter dem Begriff "Gefüge" wird im Folgenden die Anordnung der Bestandteile von Feststoffen (Festkörpern) insbesondere die Anordnung der Kristallite (Körner), Poren, der amorphen Bereiche und Korngrenzbereiche des Implantatkörpers verstanden. Ferner bezeichnet der Ausdruck "oberflächennahes Gefüge" einen Volumenbereich des Gefüges des Implantatkörpers, welcher sich von der Oberfläche bis in eine bestimmte (geringe) Tiefe des Implantatkörpers erstreckt. Dieser sich von der Oberfläche bis in eine bestimmte Tiefe erstreckende Volumenbereich des Implantatkörpers wird auch als "oberflächennah angeordnete Grenzschicht des Implantatkörpers" oder kurz als "Grenzschicht" bezeichnet. Mit dem Begriff "Kristallstruktur" wird die innerhalb eines Kristallits (Korns) vorliegende Anordnung der Atome verstanden.

Ein weiterer Vorteil des erfindungsgemäßen Verfahrens besteht darin, dass die Freisetzung von NOₓ nicht mit einer Freisetzung eines festen oder flüssigen Wirkstoffs verbunden ist. Ferner sind bislang keine Wechselwirkungen des NOₓ-Gases mit dem Material des Implantats oder Reaktionsprodukten dieses Materials bei der Degradation beobachtet worden.

Es ist außerdem von Vorteil, dass aufgrund der großen, in den Poren oder Mikrokavernen vorhandenen Oberfläche eine große Gasmenge NOₓ gebunden werden kann.

Gemäß der vorliegenden Erfindung wird die Porenstruktur mittels Wärmebehandlung des Implantatkörpers in einer Sauerstoff enthaltenden und/oder Kohlenstoff enthaltenden Atmosphäre hergestellt. Eine Wärmebehandlung ist ein besonders einfaches und kostengünstiges Verfahren, das die Voraussetzungen für die Einlagerung eines NOₓ-Gases schafft.

Es ist weiter von Vorteil, dass die Porenstruktur des Implantatkörpers als Stoffreservoir für eine unten näher beschriebene weitere Beschichtung mittels einer pharmazeutisch aktiven Substanz dienen kann, die als Nano- oder Mikropartikel eingelagert wird und beispielsweise das Knochenwachstum fördernde Substanzen wie Kalziumphosphate, temporär wirkende Kontrastmittel und/oder zellwachstumshemmende radioaktive Substanzen umfassen kann. Ferner kann in die Porenstruktur effektiv Gleitmittel zur Verringerung des Reibungskoeffizienten in einem Katheter eingelagert werden.

Eine besonders effektive Einlagerung von NOₓ-Gas in die Porenstruktur wird dadurch erreicht, dass NO-Gas unter Aufbringung eines Überdrucks von mindestens 5 bar, vorzugsweise von mindestens 8 bar in das Gefüge und/oder die Kristallstruktur eingelagert wird. Hierbei kann reines NO-Gas oder eine Mischung von NO-Gas mit anderen Gasen verwendet werden.

Es ist weiterhin von Vorteil, dass nach Schritt c) eine Beschichtung des Implantatkörpers zumindest auf einem Teil seiner behandelten Oberfläche mit einem Polymer, vorzugsweise mit einem Polymer aus der Gruppe enthaltend Magnesiumstearat, Parylene und pharmazeutisch aktive Substanzen erfolgt.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine (ggf. zusätzlich zu NOₓ vorhandene) antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, Everolimus, Biolimus A9, Deforolimus und ihre Derivate bzw. Prodrugs, bestehen.

Eine Beschichtung der mit NOₓ versehenen oberflächennahen Grenzschicht des Implantats mittels Parylene und/oder Magnesiumstearat ist von Vorteil, da die während der Herstellung vor der Aufbringung von Parylene und/oder Magnesiumstearat vorliegenden Oberflächeneigenschaften durch die darüber liegende Beschichtung erhalten d.h. buchstäblich 'eingefroren' werden. Hierdurch können die Oberflächeneigenschaften, die sonst ggf. von der Lagerungs- oder Transportdauer des Implantats bis zum Einbringen in den zu behandelnden Organismus abhängen, und damit auch die Degradationsdauer reproduzierbar und definiert eingestellt werden. Zudem kann die Freisetzung von NOₓ gesteuert werden. Dieser Effekt beruht auf der Wirkung als Diffusionsbarriere gegenüber der Permeation von Wassermolekülen und Chloridionen.

Vorteilhaft wirkt sich bei einer Beschichtung mit Parylene die hohe Spaltgängigkeit von Parylene aus, so dass auch eine tiefgehende Penetration der durch die Temper-Behandlung erzeugten Porenstruktur erfolgt. Die für Parylene, insbesondere Parylene N, charakteristischen Permeationseigenschaften für Wasser, chloridhaltige Lösungen und Wasserstoff sorgen in Verbindung mit der darunter liegenden Oberfläche für ein besonders gut steuerbares Degradationsverhalten des Implantats. Dieses zeichnet sich auch durch einen über den Implantatquerschnitt uniformen langsamen Korrosionsfortschritt aus. Zudem leistet die Parylene-Schicht einen zusätzlichen Beitrag zur Vermeidung bzw. Behinderung des Rissfortschritts bei mechanischer Belastung und verhindert partielle Schichtablösungen.

Parylene ist die Bezeichnung für vollständig lineare, teilkristalline, unvernetzt aromatische Polymere. Die verschiedenen Polymere besitzen unterschiedliche Eigenschaften und lassen sich in vier Grundtypen einteilen, nämlich Parylene C, Parylene D, Parylene N und Parylene F. Für die weitere Beschichtung nach einer tribochemischen Behandlung wird vorzugsweise Parylene N verwendet.

Mittels des erfindungsgemäßen Verfahrens kann bei der Beschichtung mit Magnesiumstearat ein Implantat hergestellt werden, das sich durch Defektfreiheit der Körperoberfläche durch nachträgliches Versiegeln auszeichnet. Lokale Fehlstellen und/oder auf der Körperoberfläche des Implantats vorhandene Poren werden wirksam vor dem Kontakt mit korrosiv wirkenden Körperflüssigkeiten geschützt. Die hydrophobe Oberflächeneigenschaft und der geringe Kristallwassergehalt des Magnesiumstearats, der auch durch einen vorzugsweise durchgeführten, sich an das Aufbringen der Magnesiumstearat-Beschichtung anschließenden Trocknungsschritt erzeugt wird, beschränken während der sich anschließenden Lagerung und dem Transport des Implantats die Diffusion von Wasser zum Grundwerkstoff des Implantatkörpers. Ebenso wird das Loslösen von Partikeln mit geringer Bindungsneigung von der Oberfläche des Implantat-Körpers beim Dilatieren verhindert. Diese Partikel verbleiben in der zähen, hoch flexiblen Magnesiumstearatschicht. Hierdurch ergibt sich eine erhöhte Hämo- beziehungsweise Biokompatibilität.

Aufgrund der Magnesiumstearat-Beschichtung des Implantat-Körpers wird in vorteilhafter Weise bewirkt, dass der Reibungskoeffizient des Implantats sinkt. Hieraus folgt, dass beim Verschieben beispielsweise eines Stents als Implantat in einem Katheter geringere Kräfte aufgewendet werden müssen. Dadurch wird im Falle eines Stents eine genauere Stentfixation ermöglicht. Zudem werden das Crimpen und die spätere Freisetzung des Implantats an dem zu behandelnden Ort vereinfacht.

In einem bevorzugten Ausführungsbeispiel des erfindungsgemäßen Verfahrens wird die Magnesiumstearat-Beschichtung mittels Eintauchen in eine Lösung aufgebracht, wobei die Lösung Magnesiumstearat und ein Lösungsmittel, vorzugsweise Aceton und/oder Isopropanol, enthält und vorzugsweise eine Temperatur zwischen etwa 10°C und dem jeweiligen Siedepunkt des Lösungsmittel aufweist. Alternativ kann die Magnesiumstearatschicht auch derart aufgebracht werden, dass die genannte magnesiumstearathaltige Lösung auf den Körper des Implantats aufgesprüht wird (spray coating). Dabei wird das Teil in einer Kammer an einem dünnen Draht aufgehängt und mittels einem rotierenden Teller (Chargenhalter) allseitig besprüht.

In einem bevorzugten Ausführungsbeispiel kann die Effektivität des Tauchprozesses durch Anlegen eines Drucks erhöht werden, der kleiner als der Umgebungsdruck, vorzugsweise kleiner als etwa 90% des Umgebungsdrucks ist, d.h. des Luftdrucks an dem Ort, an dem der Tauchprozess durchgeführt wird. Der hierbei eintretende Entgasungseffekt führt zu einer schnellen Füllung der filigranen Oberflächenstruktur des Implantats mit Magnesiumstearat. Nach einer Verweildauer von einigen Minuten in der Lösung, vorzugsweise von mindestens etwa 2 Minuten, wird der mit Magnesiumstearat beschichtete Implantat-Körper aus dem Tauchbad entfernt und im Trockenofen bei einer Temperatur, die größer ist als die Raumtemperatur, vorzugsweise größer als etwa 30°C, getrocknet. Hierbei ist es besonders bevorzugt, wenn die Trocknungstemperatur möglichst gering ist, d.h. zwischen etwa 40°C und etwa 70°C liegt, da hierdurch ein langsames Freisetzen/Abdampfen des mindestens einen Lösungsmittels führt, wodurch einen porenfreie Schicht, die Magnesiumstearat enthält, erzeugt wird.

In einem bevorzugten Ausführungsbeispiel enthält der Körper des Implantats vorzugsweise ein degradierbares metallisches Material, vorzugsweise überwiegend Eisen, insbesondere mehr als 90 Gew.% Eisen, besonders bevorzugt mindestens 99 Gew.% Eisen, insbesondere in einer Legierung. Als weitere metallische Materialien können alternativ oder zusätzlich Mangan und/oder Wolfram eingesetzt werden. Diese Implantate werden, da sie kostengünstig herstellbar sind, besonders gern für die Behandlung von Erkrankungen des menschlichen oder tierischen Organismus eingesetzt.

Die obige Aufgabenstellung wird ferner gelöst durch ein Implantat erhältlich durch eines der oben beschriebenen erfindungsgemäßen Verfahren. Ein derartiges Implantat besitzt die oben im Zusammenhang mit dem erfindungsgemäßen Herstellungsverfahren angegebenen Vorteile. Die durch die Wärmebehandlung und die NOₓ-Gas-Einlagerung entstehende Morphologie der oberflächennah angeordneten Grenzschicht und die Zusammensetzung dieser sind charakteristisch für diese Behandlung und am fertig hergestellten Implantat erkennbar.

Die obige Aufgabenstellung wird ebenfalls gelöst durch ein Implantat, insbesondere einer intraluminalen Endoprothese, mit einem Körper enthaltend ein degradierbares metallisches Material, vorzugsweise Eisen, dessen Körper eine Porenstruktur mit zumindest teilweise geschlossenen Poren (Leerstellenstruktur) in einem Teil seines oberflächennahen Gefüges aufweist, welche ein NOₓ-haltiges Gas enthält. Auch für dieses erfindungsgemäße Implantat ergeben sich die oben bereits erläuterten Vorteile.

In einem bevorzugten Ausführungsbeispiel weisen die Poren der Porenstruktur einen Durchmesser von maximal etwa 1 µm auf.

Es ist ferner von Vorteil, wenn sich die Poren der Porenstruktur bis in eine Tiefe von maximal etwa 15 µm, vorzugsweise von etwa 10 µm bis etwa 15 µm, von der Oberfläche des Implantatkörpers gemessen erstrecken. In einem bevorzugten Ausführungsbeispiel beträgt die Konzentration des NOₓ-Gases im oberflächennahen Gefüge des Implantatkörpers, das im oberflächennahen Grenzbereich vorliegt, etwa 10 Vol.%. Die Tiefe von ca. 15 µm wird bei einer vergleichsweise praktikablen Verfahrensdauer von 2 bis 4 Stunden bei einem Behandlungsdruck von 10 bar in einer NOₓ-Atmosphäre erreicht. Diese Eindringtiefe und das damit verbundene Volumen an eingelagertem NOₓ (etwa 10 Vol.%) im Gefüge reichen aus, um über einen Zeitraum von mehreren Tagen bis zu 4 Wochen nach dem Einbringen des Implantats an die zu behandelnde Stelle antiinflammatorische Wirkungen zu erzielen. Erst nach Ablauf dieses Zeitraums ist die Degradation so weit fortgeschritten, dass Bauteilbereiche, die keine erhöhte Konzentration an NOₓ mehr aufweisen, mit Körperflüssigkeit (Elektrolyt) in Berührung kommen.

Längere Behandlungszeiten und/oder höhere Verfahrensdrücke würden zu Konzentrationen an NOₓ führen, die eine mechanische Schädigung der oberflächennahen Gefügebereiche des behandelten Implantats hervorrufen könnten. Eine solche Schädigung könnte zu einer zu hohen Versprödung und einer damit verbundenen Bildung instabiler Risse führen, so dass unter Umständen der Zusammenhalt des inneren Gefüges des Implantats verloren gehen könnte.

Ebenfalls ist bevorzugt, wenn die Oberfläche des Implantatkörpers zumindest teilweise eine Beschichtung aufweist, welche ein Polymer, vorzugsweise ein Polymer aus der Gruppe enthaltend Magnesiumstearat, Parylene und pharmazeutische aktive Substanzen enthält. Hierbei liegen bevorzugte Schichtdicken der Parylene-Beschichtung zwischen etwa 0,5 und etwa 2,0 µm. Die bevorzugte Dicke der Magnesiumstearat-Beschichtung beträgt etwa 0,5 µm bis etwa 2,0 µm, vorzugsweise etwa 0,7 µm bis etwa 1,0 µm. Die Konzentration des Magnesiumstearats in der Beschichtung liegt dabei etwa zwischen 80 Gew.% und 100 Gew.%.

Durch die Beschichtung mittels Magnesiumstearat und/oder Parylene kann die Degradationszeit des Implantats in weiten Grenzen entsprechend des jeweiligen Einsatzzwecks des Implantats variiert und definiert eingestellt werden.

Das erfindungsgemäße Verfahren bzw. das erfindungsgemäße Implantat wird nachfolgend in Beispielen anhand von Figuren erläutert.

Es zeigen schematisch:
Fig. 1 ein Strebe eines erfindungsgemäßen Implantats im Querschnitt und
Fig. 2 einen vergrößerten Ausschnitt des in Figur 1 dargestellten Ausführungsbeispiels.

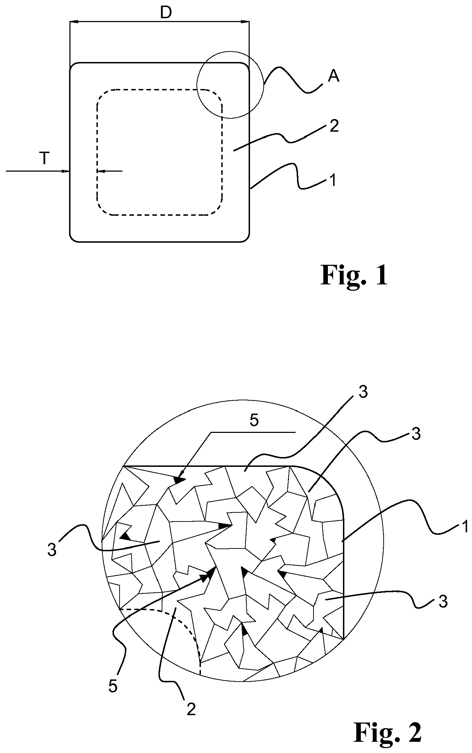

In Figur 1 ist der Querschnitt einer erfindungsgemäßen Stent-Strebe 1 dargestellt, welche in einem äußeren, von der Oberfläche der Stent-Strebe ausgehenden, oberflächennahen Grenzbereich 2, welcher sich bis in eine Tiefe T erstreckt, eine zumindest teilweise geschlossene Porenstruktur aufweist, in der NOₓ mit einer Konzentration von etwa 10 Vol.% eingelagert ist. Die Abmessung T des Bereichs 2 mit NOₓ beträgt etwa 10 µm bis 15 µm. Der Durchmesser D der Stent-Strebe 1 beträgt etwa 100 µm.

An dieser Stelle soll ausdrücklich darauf hingewiesen werden, dass die oben angegebene NOₓ-Konzentration in dem am weitesten innen liegenden Teil des Bereichs 2 (mit einer gestrichelten Linie gekennzeichnet) langsam in Richtung der Ausgangszusammensetzung des Stent-Streben-Materials abnimmt, so dass die Konzentration von NOₓ sich dort langsam ändert und nicht sprunghaft abnimmt. Die Zusammensetzung des Stent-Streben-Materials im Inneren der Strebe (innerhalb der gestrichelten Linie) entspricht im Wesentlichen der Ausgangszusammensetzung vor Anwendung des erfindungsgemäßen Verfahrens. Figur 2 zeigt einen vergrößerten Ausschnitt des in Figur 1 dargestellten Querschnitts im Bereich A. In dem Ausschnitt A der Strebe 1 sind Körner 3 des Gefüges des Stent-Streben-Materials erkennbar. In Mikroporen des Gefüges, in denen zuvor COₓ-Verbindungen vorhanden waren, die insbesondere in den mit dem Bezugszeichen 5 und schwarz markierten Bereiche der Korngrenzen, die insbesondere in den Korngrenz-Zwickel angeordnet sind, ist nun NOₓ eingelagert. Die Einlagerung von NOₓ in die Porenstruktur erfolgte gemäß eines der nachfolgend beschriebenen erfindungsgemäßen Verfahren. Das NOₓ besitzt insbesondere antiinflammatorische Wirkung bei der Degradation des erfindungsgemäßen Stents nach Einsetzen des Stents in das zu behandelnde Gefäß.

### 1. Beispiel

Ein Stent mit einem Körper, der aus einer Eisenbasis-Legierung mit mindestens 98 Gew.% Eisen, beispielsweise den Legierungen C10, C15 oder C20, besteht, wird bei einer Temperatur von etwa 850°C in einem Kohlenstoff enthaltenden Trägergas (z.B. ein Gemisch aus 60 Vol % Methanol und 40 Vol % Stickstoff) für etwa 1,5 Stunden reduzierend wärmebehandelt. Während dieses in der Regel als Gasaufkohlung bezeichneten Prozesses zerfällt gasförmiges Kohlenmonoxid an der Oberfläche des behandelten Teils zu atomarem Kohlenstoff und Sauerstoff. Die Kohlenstoffatome werden vom Metall absorbiert und diffundieren im Folgenden in die Stentoberfläche hinein. Je nach Behandlungsdauer kann die Diffusionstiefe und der Kohlenstoff-Gradient in der Matrix der Eisenlegierung variiert werden. Variiert werden können insbesondere die Parameter Gasgemisch, Zeit, Temperatur und Konzentration des Kohlenstoff enthaltenden Gases (Argon kann als zusätzliches Inertgas das Angebot an C und damit die C-Diffusion verringern). Mit Erhöhung des C-Angebotes, der Zeit und der Temperatur steigt die C-Konzentration in dem oberflächennahen Gefügebereich. Der Kohlenstoff dringt auch tiefer in das Gefüge ein. Anschließend wird der Stent in einer sauerstoffhaltigen, d.h. oxidierenden, Atmosphäre, beispielsweise mit 50 Vol. % Stickstoff und 50 Vol. % Sauerstoff, bei einer Temperatur von etwa 800°C über 1,5 h Stunden geglüht. Dabei entstehen Kohlenstoffoxide (auf Grund des Überangebotes an Sauerstoff vorwiegend CO₂). Diese Gase entweichen aus dem oberflächennah angeordneten Gefüge des Stentkörpers. Die so erhaltene randentkohlte Zone weist eine Tiefe T von 10 bis 15 µm auf.

Eine Beschleunigung des Entgasungseffekts kann durch ein nach dem oxidierenden Prozess durchgeführtes Glühen bei etwa 800°C im Vakuum erzielt werden. Beispielsweise wird ein Vakuum von mindestens 10⁻⁴ Pa über einen Zeitraum von mindestens drei Stunden gehalten, so dass die in dem Stentkörper enthaltenen COₓ-Verbindungen aus den Hohlräumen (Poren) entweichen. Des Weiteren bewirkt die Vakuumbehandlung eine Reduktion der sich an der Oberfläche ausgebildeten und als Diffusionsbarriere wirkenden Oxidschicht und die COₓ-Verbindungen können somit schneller aus der Stentoberfläche entweichen.

Durch die Wärmebehandlung (thermische Behandlung) wird eine mit Mikrokarvenen (Poren) durchsetzte, oberflächennahe poröse Struktur in dem Stentkörper erzeugt. In der gleichen Behandlungskammer, in der bereits die Reduktion, die Oxidation und die Vakuumglühung durchgeführt wurden, erfolgt nun eine Belüftung mit NO-Gas. Dieses wird mit einem Überdruck von bis zu ca. 10 bar in die Hohlräume gedrückt. Durch diese Behandlung reichern sich die Mikrokavernen mit NOₓ-Gas an.

Der Stent wird nach Abschluss der NOₓ-Beladung aus der Behandlungskammer entnommen und anschließend in einem Katheter montiert. Eine nun folgende Lagerung des in einem Katheter montierten Stents an Luft oder unter Schutzgas bei Normaldruck bewirkt lediglich ein geringfügiges Entweichen der NOₓ-Verbindungen aus dem Stentkörper.

Wie nachfolgend beschrieben wird, kann die Oberfläche des Stents anschließend mit einem abbaubaren oder einem nichtabbaubaren Polymer versiegelt werden.

Im Rahmen der Behandlung mit dem so hergestellten Stent entstehen bei mechanischer Beanspruchung, bspw. beim Dilatieren des Stents, in den am stärksten plastisch verformten Zonen des Stents Risse. Diese reichen bis in die Poren und befördern somit die Freisetzung des eingelagerten NOₓ-Gases.

### 2. Beispiel

Analog zu Beispiel 1, wobei das Material des Stents die folgende Zusammensetzung aufweist (Eisenbasislegierung mit Mn als Stentmaterial): Fe- Legierung mit 82 Gew.% Fe, 15 Gew.% Mn und 3 Gew.% Pd (Fe82Mn15Pd3). Als Trägergas zur Hohlraumerzeugung wird Schwefelwasserstoff eingesetzt.

### 3. Beispiel

Bei allen nach den Beispielen 1 und 2 hergestellten Stents kann als finaler Schritt eine Beschichtung mit Parylene C, Magnesiumstearat und/oder einer pharmazeutisch aktiven Substanz erfolgen.

Die Beschichtung mit Parylene C erfolgt aus der Gasphase. Nach ca. einer halben Stunde Beschichtungszeit wird eine Schichtdicke von ca. 0,5 µm erzielt.

Mittels einer Parylene-Beschichtung kann ein temporärer Korrosionsschutz erreicht werden. Der Oberflächenzustand wird "eingefroren". Es erfolgt somit keine unkontrollierte selbstständig verlaufende Degradation vor dem Verbringen der Endoprothese an den Einsatzort.

Das gleiche Ziel wird mit der nachfolgend beschriebenen Magnesiumstearatbeschichtung verfolgt. Nach der Durchführung der Ausführungsbeispiele 1 bis 3 und einer daran anschließenden Trocknung, wird die Endoprothese auf einen Kunststofffaden (z.B. Polyamid) aufgehängt und anschließend in die Lösung zur Auftragung des Magnesiumstearats getaucht. Die Lösung besteht z.B. aus neun Teilen hochreinem Aceton oder Isopropanol und einem Teil Magnesiumstearat. Der Tauchvorgang erfolgt bei Raumtemperatur in einem evakuierbaren Exsikkator. In diesem wird ein Unterdruck von ca. 100 mbar mittels einer Pumpe erzeugt. Hierdurch werden die filigranen und durch die vorangegangene plasmachemische Vorbehandlung entstandenen mikroporösen Oberflächenstrukturen bzw. die Hinterschneidungen und kompliziert geformten Strukturen effektiv von Restgas befreit. Dadurch kann in der Lösung eine vollständige Bedeckung der Stentoberfläche durch das Magnesiumstearat erfolgen, das auch in die Oberflächenstrukturen und Hinterschneidungen eindringt. Nach einer Verweildauer von etwa 3 Minuten in dem Tauchbad wird der Exsikkator belüftet, das Implantat aus dem Tauchbad entnommen und anschließend in einem Umluftschrank, immer noch am Kunststofffaden hängend, bei einer Temperatur von 60°C getrocknet. Die Schichtdicke der so erhaltenen Magnesiumstearat-Beschichtung liegt dabei im Bereich von etwa 0,5 bis etwa 10 µm.

Bedingt durch den im Exsikkator vorliegenden Unterdruck wird das Magnesiumstearat sehr gleichmäßig auf der Oberfläche abgeschieden. Eine geringe Trocknungstemperatur bewirkt in vorteilhafter Weise ein langsames Freisetzen/Abdampfen der Lösungsmittel der Tauch-Lösung, so dass eine porenfreie Magnesiumstearatschicht entsteht. Handelt es sich bei dem so hergestellten Implantat um einen Stent, so kann der mit der ersten Schicht und der Zwischenschicht versehene Körper anschließend mit einem Katheter komplettiert und einer Strahlensterilisation unterzogen werden.

Analog zu der Herstellung der Parylene- oder Magnesiumstearat-Beschichtung kann die Oberfläche des Implantats alternativ oder zusätzlich mit einer pharmazeutisch aktiven Substanz beschichtet werden. Bevorzugte Substanzen sind oben in der Beschreibung angegeben.

## Patentansprüche

1. Verfahren zur Herstellung eines Implantats (1), insbesondere einer intraluminalen Endoprothese, mit einem Körper enthaltend metallisches Material, vorzugsweise Eisen, umfassend die folgenden Schritte:
a) Bereitstellen des Körpers des Implantats (1),
b) Herstellung einer Porenstruktur mit zumindest teilweise geschlossenen Poren in einem Teil des oberflächennahen Gefüges des Implantatkörpers,
c) Einlagerung von NOₓ in die Hohlräume der Porenstruktur,
**dadurch gekennzeichnet, dass** die Porenstruktur mittels Wärmebehandlung des Implantatkörpers in einer Sauerstoff enthaltenden und/oder Kohlenstoff enthaltenden Atmosphäre hergestellt wird.

2. Verfahren nach Anspruch 1, **dadurch gekennzeichnet, dass** die Porenstruktur mittels Wärmebehandlung des Implantatkörpers in einer Kohlenstoff enthaltenden Atmosphäre und mittels Wärmebehandlung in einer Sauerstoff enthaltenden Atmosphäre hergestellt wird.

3. Verfahren nach einem der vorhergehenden Ansprüche, **dadurch gekennzeichnet, dass** das NOₓ-Gas unter Aufbringung eines Überdrucks von mindestens 5 bar, vorzugsweise von mindestens 8 bar, in das Gefüge und/oder die Kristallstruktur eingelagert wird.

4. Verfahren nach einem der vorhergehenden Ansprüche, **dadurch gekennzeichnet, dass** nach Schritt c) eine Beschichtung des Implantatkörpers zumindest auf einem Teil seiner Oberfläche mit einem Polymer, oder Magnesiumstearat oder Parylene oder einer pharmazeutisch aktiven Substanzen erfolgt.

5. Verfahren nach einem der vorhergehenden Ansprüche, **dadurch gekennzeichnet, dass** der Körper des Implantats überwiegend Eisen aufweist, vorzugsweise mehr als 90 Gew.%, besonders bevorzugt mehr als 99 Gew.% Eisen, insbesondere in einer Legierung, enthält.

6. Implantat (1), insbesondere eine intraluminalen Endoprothese, mit einem Körper enthaltend ein degradierbares metallisches Material, vorzugsweise Eisen, **dadurch gekennzeichnet, dass** der Implantatkörper eine Porenstruktur mit zumindest teilweise geschlossenen Poren in einem Teil seines oberflächennahen Gefüges aufweist, welche in ihren Hohlräumen NOₓ enthält.

7. Implantat nach Anspruch 6, **dadurch gekennzeichnet, dass** die Poren der Porenstruktur einen Durchmesser von maximal etwa 1 µm aufweisen.

8. Implantat nach einem der Ansprüche 6 oder 7, **dadurch gekennzeichnet, dass** die das NOₓ aufnehmenden Hohlräume der Porenstruktur sich bis in eine Tiefe (T) von maximal etwa 15 µm, gemessen von der Oberfläche des Implantatkörpers aus, erstrecken.

9. Implantat nach einem der Ansprüche 6 bis 8, **dadurch gekennzeichnet, dass** die Konzentration von NOₓ im oberflächennahen Gefüge des Implantatkörpers etwa 10 Vol. % beträgt.

10. Implantat nach einem der Ansprüche 6 bis 9, **dadurch gekennzeichnet, dass** die Oberfläche des Implantatkörpers zumindest teilweise eine Beschichtung aufweist, welche ein Polymer, oder Magnesiumstearat oder Parylene, und/oder eine pharmazeutisch aktive Substanz enthält.

## Claims

1. A process to produce an implant (1), especially an intraluminal endoprosthesis, with a body containing metal material, preferably iron, especially an iron alloy, this process comprising the following steps:
a) Preparing the body of the implant (1);
b) Producing a pore structure with at least some closed pores in one part of the near-surface structure of the implant body;
c) Incorporating NOx into the hollow spaces of the pore structure;
**characterized in that** the pore structure is produced by means of heat treatment of the implant body in an oxygen-containing and/or carbon-containing atmosphere.

2. A process according to claim 1 **characterized in that** the pore structure is produced by means of heat treatment of the implant body in a carbon-containing atmosphere and by means of heat treatment in an oxygen-containing atmosphere.

3. A process according to any one of the preceding claims, **characterized in that** the NOx gas is incorporated into the structure and/or the crystal structure while an excess pressure of at least 5 bar, preferably at least 8 bar, is applied.

4. A process according to any one of the preceding claims, **characterized in that** after step c) at least part of the surface of the implant body is coated with a polymer or magnesium stearate or parylene or a pharmaceutically active substance.

5. A process according to any one of the preceding claims, **characterized in that** the body of the implant contains predominantly iron, preferably more than 90 weight % iron, especially preferably at least 99 weight % iron, especially in an alloy.

6. An implant (1), especially an intraluminal endoprosthesis, with a body containing a degradable metal material, preferably iron, **characterized in that** the implant body has a pore structure with at least some closed pores in one part of its near-surface structure, this pore structure containing NOx in its hollow spaces.

7. An implant according to claim 6, **characterized in that** the pores of the pore structure have a maximum diameter of about 1 µm.

8. An implant according to one of claims 6 or 7, **characterized in that** the NOx holding hollow spaces of the pore structure extend down to a maximum depth (T) of about 15 µm, measured from the surface of the implant body.

9. An implant according to any one of the claims 6 through 8, **characterized in that** the concentration o NOx in the near-surface structure of the implant body is about 10 volume %.

10. An implant according to any one of the claims 6 through 9, **characterized in that** at least part of the surface of the implant body has a coating that contains a polymer or magnesium stearate or parylene and/or a pharmaceutically active substance.

## Revendications

1. Procédé de fabrication d'un implant (1), notamment d'une endoprothèse intraluminale, doté d'un corps contenant un matériau métallique, de préférence du fer, comprenant les étapes suivantes :
a) de fourniture du corps de l'implant (1),
b) de fabrication d'une structure poreuse avec des pores au moins partiellement fermés dans une partie de la texture du corps d'implant proche de la surface,
c) d'incorporation de NOₓ dans les corps creux de la structure poreuse,
**caractérisé en ce que** la structure poreuse est fabriquée au moyen d'un traitement thermique du corps d'implant dans une atmosphère contenant de l'oxygène et/ou contenant du carbone.

2. Procédé selon la revendication 1, **caractérisé en ce que** la structure poreuse est fabriquée au moyen d'un traitement thermique du corps d'implant dans une atmosphère contenant du carbone et au moyen d'un traitement thermique contenant de l'oxygène.

3. Procédé selon l'une des revendications précédentes, **caractérisé en ce que** le gaz NOₓ est incorporé dans la texture et/ou la structure cristalline moyennant l'application d'une surpression d'au moins 5 bar, de préférence d'au moins 8 bar.

4. Procédé selon l'une des revendications précédentes, **caractérisé en ce qu'**après l'étape c), il y a un revêtement du corps d'implant, au moins sur une partie de sa surface, avec un polymère, ou du stéarate de magnésium ou des parylènes, ou avec une substance pharmaceutiquement active.

5. Procédé selon l'une des revendications précédentes, **caractérisé en ce que** le corps de l'implant présente principalement du fer, de préférence plus de 90 % en poids, de manière particulièrement préférée, plus de 99 % en poids de fer, en particulier dans un alliage.

6. Implant (1), notamment endoprothèse intraluminale, doté d'un corps contenant un matériau métallique dégradable, de préférence, du fer, **caractérisé en ce que** le corps d'implant présente une structure poreuse avec des pores au moins partiellement fermés dans une partie de sa texture proche de la surface, laquelle contient du NOₓ dans ses corps creux.

7. Implant selon la revendication 6, **caractérisé en ce que** les pores de la structure poreuse présentent un diamètre d'au maximum environ 1 µm.

8. Implant selon l'une des revendications 6 ou 7, **caractérisé en ce que** les corps creux de la structure poreuse recevant le NOₓ s'étendent jusqu'à une profondeur (T) d'au maximum environ 15 µm, mesurée à partir de la surface du corps d'implant.

9. Implant selon l'une des revendications 6 à 8, **caractérisé en ce que** la concentration de NOₓ dans la texture proche de la surface du corps d'implant est d'environ 10 % en volume.

10. Implant selon l'une des revendications 6 à 9, **caractérisé en ce que** la surface du corps d'implant présente au moins partiellement un revêtement, lequel contient un polymère, ou du stéarate de magnésium ou des parylènes, et/ou une substance pharmaceutiquement active.
